(19) Europäisches Patentamt
European Patent Office
Office européen des brevets

(11) EP 1 526 186 B1

(12) **EUROPEAN PATENT SPECIFICATION**

(45) Date of publication and mention of the grant of the patent:
**22.10.2008 Bulletin 2008/43**

(51) Int Cl.:
***C12Q 1/68*** *(2006.01)*

(21) Application number: **04251933.0**

(22) Date of filing: **31.03.2004**

(54) **Colorectal cancer prognostics**

Prognose von Kolorektalem Krebs

Pronostic de cancer colorectal

(84) Designated Contracting States:
**AT BE BG CH CY CZ DE DK EE ES FI FR GB GR HU IE IT LI LU MC NL PL PT RO SE SI SK TR**

(30) Priority: **31.03.2003 US 403449**

(43) Date of publication of application:
**27.04.2005 Bulletin 2005/17**

(73) Proprietor: **Veridex LLC**
**Raritan, NJ 08669 (US)**

(72) Inventor: **Wang, Yixin**
**San Diego, CA 92130 (US)**

(74) Representative: **Mercer, Christopher Paul et al**
**Carpmaels & Ransford**
**43-45 Bloomsbury Square**
**London WC1A 2RA (GB)**

(56) References cited:
**EP-A- 1 349 104** **EP-A- 1 355 150**
**WO-A-00/19206** **WO-A-01/94629**
**US-A1- 2004 191 782**

- **ZHANG L ET AL: "Gene expression profiles in normal and cancer cells" SCIENCE, AMERICAN ASSOCIATION FOR THE ADVANCEMENT OF SCIENCE,, US, vol. 276, 23 May 1997 (1997-05-23), pages 1268-1272, XP002083785 ISSN: 0036-8075**
- **HEGDE ET AL: "Identification of tumor markers in models of human colorectal cancer using a 19200-element complementary DNA microarray" CANCER RESEARCH, AMERICAN ASSOCIATION FOR CANCER RESEARCH, BALTIMORE, MD, US, vol. 61, no. 21, 1 November 2001 (2001-11-01), pages 7792-7797, XP002210615 ISSN: 0008-5472**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 526 186 B1

## Description

## BACKGROUND

This invention relates to prognostics for colorectal cancer based on the gene expression profiles of biological samples.

Colorectal cancer is a heterogenous disease with complex origins. Once a patient is treated for colorectal cancer, the likelihood of a recurrence is related to the degree of tumor penetration through the bowel wall and the presence or absence of nodal involvement. These characteristics are the basis for the current Staging system defined by Duke's classification. Duke's A disease is confined to submucosa layers of colon or rectum. Duke's B tumor invades through muscularis propria and could penetrate the wall of colon or rectum. Duke's C disease includes any degree of bowel wall invasion with regional lymph node metastasis.

Surgical resection is highly effective for early stage colorectal cancers, providing cure rates of 95% in Duke's A and 75% in Duke's B patients. The presence of positive lymph node in Duke's C disease predicts a 60% likelihood of recurrence within five years. Treatment of Duke's C patients with a postsurgical course of chemotherapy reduces the recurrence rate to 40%-50%, and is now the standard of care for Duke's C patients. Because of the relatively low rate of reoccurrence, the benefit of postsurgical chemotherapy in Duke' B has been harder to detect and remains controversial. However, the Duke's B classification is imperfect as approximately 20 - 30% of these patients behave more like Duke's and relapse within a 5 year timeframe. There is clearly a need to identify better prognostic factors than nodal involvement for guiding selection of Duke's B into those that are likely to relapse and those that will survive.

WO 00/19206 discloses a method to predict high risk recurrence of Dukes's B staged colorectal cancer patients using YKL-40 levels as a marker for recurrence.

WO 01/94629 discloses the combination of sequences 1 and 3 and their use in the assessment of colorectal cancer status.

## SUMMARY OF THE INVENTION

The invention is a method of assessing the likelihood of a recurrence of colorectal cancer in a patient diagnosed with or treated for colorectal cancer. The method involves the analysis of a gene expression profile.

In one aspect of the invention, the gene expression profile includes at least three genes.

In another aspect of the invention, the gene expression profile includes at least four genes.

In yet another aspect of the invention, kits include reagents for conducting the gene expression analysis prognostic of colorectal caner recurrence.

## BRIEF DESCRIPTION OF THE DRAWINGS

Fig. 1 is a plot of the intensity (y-axis) of the measurement of Homo sapiens fatty acid binding protein gene 1 in patient samples (x-axis). Greater intensity indicates greater gene expression showing that these genes are down regulated in relapsing patients.

Fig. 2 is a plot of the intensity (y-axis) of the measurement of Human intestinal peptide associated transporter gene in patient samples (x-axis). Greater intensity indicates greater gene expression showing that these genes are down regulated in relapsing patients.

Fig. 3a is a plot of the intensity (y-axis) of the measurement of MHC class II antigen (HLA-DRB1) gene in patient samples (x-axis). Greater intensity indicates greater gene expression showing that these genes are down regulated in relapsing patients.

Fig. 3b is a plot of the intensity (y-axis) of the measurment of immunoglobin-like transcript 5 protein gene in patient samples (x-axis). Greater intensity indicates greater gene expression showing that these genes are down regulated in relapsing patients.

Fig. 4 is a standard Kaplan-Meier Plot constructed from the patient data as a training set as described in the Examples.

Fig. 5 is a standard Kaplan-Meier Plot constructed from the patient data as a testing set as described in the Examples.

Fig. 6 is a standard Kaplan-Meier Plot constructed from all of the patient data as described in the Examples.

Fig. 7. is a standard ROC curve.

## DETAILED DESCRIPTION

The mere presence or absence of particular nucleic acid sequences in a tissue sample has only rarely been found to have diagnostic or prognostic value. Information about the expression of various proteins, peptides or mRNA, on the other hand, is increasingly viewed as important. The mere presence of nucleic acid sequences having the potential to express proteins, peptides, or mRNA ( such sequences referred to as "genes") within the genome by itself is not determinative of whether a protein, peptide, or mRNA is expressed in a given cell. Whether or not a given gene capable of expressing proteins, peptides, or mRNA does so and to what extent such expression occurs, if at all, is determined by a variety of complex factors. Irrespective of difficulties in understanding and assessing these factors, assaying gene expression can provide useful information about the occurrence of important events such as tumerogenesis, metastasis, apoptosis, and other clinically relevant phenomena. Relative indications of the degree to which genes are active or inactive can be found in gene expression profiles. The gene expression profiles of this invention are used to provide a prognosis and treat patients for colorectal cancer.

Sample preparation requires the collection of patient samples. Patient samples used in the inventive method are those that are suspected of containing diseased cells such as epithelial cells taken from a colon sample or from surgical margins. One useful technique for obtaining suspect samples is Laser Capture Microdisection (LCM). LCM technology provides a way to select the cells to be studied, minimizing variability caused by cell type heterogeneity. Consequently, moderate or small changes in gene expression between normal and cancerous cells can be readily detected. In a preferred method, the samples comprise circulating epithelial cells extracted from peripheral blood. These can be obtained according to a number of methods but the most preferred method is the magnetic separation technique described in U.S. Patent 6,136,182 assigned to Immunivest Corp. Once the sample containing the cells of interest has been obtained, RNA is extracted and amplified and a gene expression profile is obtained, preferably via micro-array, for genes in the appropriate portfolios.

Preferred methods for establishing gene expression profiles include determining the amount of RNA that is produced by a gene that can code for a protein or peptide. This is accomplished by reverse transcriptase PCR (RT-PCR), competitive RT-PCR, real time RT-PCR, differential display RT-PCR, Northern Blot analysis and other related tests. While it is possible to conduct these techniques using individual PCR reactions, it is best to amplify complimentary DNA (cDNA) or complimentary RNA (cRNA) produced from mRNA and analyze it via microarray. A number of different array configurations and methods for their production are known to those of skill in the art and are described in U.S. Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637.

Microarray technology allows for the measurement of the steady-state mRNA level of thousands of genes simultaneously thereby presenting a powerful tool for identifying effects such as the onset, arrest, or modulation of uncontrolled cell proliferation. Two microarray technologies are currently in wide use. The first are cDNA arrays and the second are oligonucleotide arrays. Although differences exist in the construction of these chips, essentially all downstream data analysis and output are the same. The product of these analyses are typically measurements of the intensity of the signal received from a labeled probe used to detect a cDNA sequence from the sample that hybridizes to a nucleic acid sequence at a known location on the microarray. Typically, the intensity of the signal is proportional to the quantity of cDNA, and thus mRNA, expressed in the sample cells. A large number of such techniques are available and useful. Preferred methods for determining gene expression can be found in US Patents 6,271,002 to Linsley, et al.; 6,218,122 to Friend, et al.; 6,218,114 to Peck, et al.; and 6,004,755 to Wang, et al..

Analysis of the expression levels is conducted by comparing such intensities. This is best done by generating a ratio matrix of the expression intensities of genes in a test sample versus those in a control sample. For instance, the gene expression intensities from a diseased tissue can be compared with the expression intensities generated from normal tissue of the same type (e.g., diseased colon tissue sample vs. normal colon tissue sample). A ratio of these expression intensities indicates the fold-change in gene expression between the test and control samples.

Gerie expression profiles can also be displayed in a number of ways. The most common method is to arrange a raw fluorescence intensities or ratio matrix into a graphical dendogram where columns indicate test samples and rows indicate genes. The data is arranged so genes that have similar expression profiles are proximal to each The expression ratio for each gene is visualized as a color. For example, a ratio less than one (indicating down-regulation) may appear in the blue portion of the spectrum while a ratio greater than one (indicating up-regulation) may appear as a color in the red portion of the spectrum. Commercially available computer software programs are available to display such data including "GENESPRINT" from Silicon Genetics, Inc. and "DISCOVERY" and "INFER" software from Partek, Inc..

Modulated genes used in the methods of the invention are described in the Examples. The genes that are differentially expressed are either up regulated or down regulated in patients with a relapse of colon cancer relative to those with a relapse. Up regulation and down regulation are relative terms meaning that a detectable difference (beyond the contribution of noise in the system used to measure it) is found in the amount of expression of the genes relative to some baseline. In this case, the baseline is the measured gene expression of a non-relapsing patient. The genes of interest in the diseased cells (from the relapsing patients) are then either up regulated or down regulated relative to the baseline level using the same measurement method. Diseased, in this context, refers to an alteration of the state of a body that interrupts or disturbs, or has the potential to disturb, proper performance of bodily functions as occurs with the uncontrolled proliferation of cells. Someone is diagnosed with a disease when some aspect; of that person's genotype or phenotype is consistent with the presence of the disease. However, the act of conducting a diagnosis or prognosis includes the determination of disease/status issues such as determining the likelihood of relapse and therapy monitoring. In therapy monitoring, clinical judgments are made regarding the effect of a given course of therapy by comparing the expression of genes overtime to determine whether the gene expression profiles have changed or are changing to patterns more consistent with normal tissue.

Preferably, levels of up and down regulation are distinguished based on fold changes of the intensity measurements of hybridized microarray probes. A 2.0 fold difference is preferred for making such distinctions or a p-value less than .05. That is, before a gene is said to be differentially expressed in diseased/relapsing versus normal/non-relapsing cells, the diseased cell is found to yield at least 2 more, or 2 times less intensity than the normal cells. The greater the fold difference, the more preferred is use of the gene as a diagnostic or prognostic tool. Genes selected for the gene expression profiles of the instant invention have expression levels that result in the generation of a signal that is distinguishable from those of the normal or non-modulated genes by an amount that exceeds background using clinical laboratory instrumentation.

Statistical values can be used to confidently distinguish modulated from non-modulated genes and noise. Statistical tests find the genes most significantly different between diverse groups of samples. The Student's t-test is an example of a robust statistical test that can be used to find significant differences between two groups. The lower the p-value, the more compelling the evidence that the gene is showing a difference between the different groups. Nevertheless, since microarrays measure more than one gene at a time, tens of thousands of statistical tests may be asked at one time. Because of this, one is unlikely to see small p-values just by chance and adjustments for this using a Sidak correction as well as a randomization/permutation experiment can be made. A p-value less than .05 by the t-test is evidence that the gene is significantly different. More compelling evidence a p-value less then .05 after the Sidak correction is factored in. For a large number of samples in each group, a p-value less than 0.05 after the randomization/permutation test is the most compelling evidence of a significant difference.

Another parameter that can be used to select genes that generate a signal that is greater than that of the non-modulated gene or noise is the use of a measurement of absolute signal difference. Preferably, the signal generated by the modulated gene expression is at least 20% different than those of the normal or non-modulated gene (on an absolute basis). It is even more preferred that such genes produce expression patterns that are at least 30% different than those of normal or non-modulated genes.

Genes can be grouped so that information obtained about the set of genes in the group provides a sound basis for making a clinically relevant judgment such as a diagnosis, prognosis, or treatment choice. These sets of genes make up the portfolios of the invention. In this case, the judgments supported by portfolios involve colorectal cancer. As with most diagnostic markers, it is often desirable to use the fewest number of markers sufficient to make a correct medical judgment. This prevents a delay in treatment pending further analysis as well inappropriate use of time and resources.

Preferably, portfolios are established such that the combination of genes in the portfolio exhibit improved sensitivity and specificity relative to individual genes or randomly selected combinations of genes. In the context of the instant invention, the sensitivity of the portfolio can be reflected in the fold differences exhibited by a gene's expression in the diseased state relative to the normal state. Specificity can be reflected in statistical measurements of the correlation of the signaling of gene expression with the condition of interest. For example, standard deviation can be a used as such a measurement. In considering a group of genes for inclusion in a portfolio, a small standard deviation in expression measurements correlates with greater specificity. Other measurements of variation such as correlation coefficients can also be used in this capacity.

A preferred method of establishing gene expression portfolios is through the use of optimization algorithms such as the mean variance algorithm widely used in establishing stock portfolios. This method is described in detail in the patent application entitled "Portfolio Selection" by Tim Jatkoe, et, al., filed on March 21,2003. Essentially, the method calls for the establishment of a set of inputs (stocks in financial applications expression as measured by intensity here) that will optimize the return (e.g., signal that is generated) one receives for using it while minimizing the variability of the return. Many commercial software programs are available to conduct such operations. "Wagner Associates Mean-Variance Optimization Application", referred to as "Wagner Software" throughout this specification, is preferred. This software uses functions from the "Wagner Associates Mean-Variance Optimization Library" to determine an efficient frontier and optimal portfolios in the Markowitz sense.is preferred.

Use of this type of software requires that microarray data be transformed so that it can be treated as an input in the way stock return and risk measurements are used when the software is used for its intended financial analysis purposes. For example, when Wagner Software is employed in conjunction with microarray intensity measurements the following data transformation method is employed.

Genes are first pre-selected by identifying those genes whose expression shows at least some minimal level of differentiation. The preferred pre-selection process is conducted as follows. A baseline class is selected. Typically, this will comprise genes from a population that does not have the condition of interest. For example, if one were interested in selecting a portfolio of genes that are diagnostic for relapsing colon cancer, samples from patients without relapses can be used to make the baseline class. Once the baseline class is selected, the arithmetic mean and standard deviation is calculated for the indicator of gene expression of each gene for baseline class samples. This indicator is typically the fluorescent intensity of a microarray reading. The statistical data computed is then used to calculate a baseline value of (X*Standard Deviation + Mean) for each gene. This is the baseline reading for the gene from which all other samples will be compared. X is a stringency variable selected by the person formulating the portfolio. Higher values of X are more stringent than lower. Preferably, X is in the range of .5 to 3 with 2 to 3 being more preferred and 3 being most preferred.

Ratios between each experimental sample (those displaying the condition of interest) versus baseline readings are then calculated. The ratios are then transformed to base 10 logarithmic values for ease of data handling by the software. This enables down regulated genes to display negative values necessary for optimization according to the Markman mean-variance algorithm using the Wagner Software.

The preprocessed data comprising these transformed ratios are used as inputs in place of the asset return values that are normally used in the Wagner Software when it is used for financial analysis purposes.

Once an efficient frontier is formulated, an optimized portfolio is selected for a given input level (return) or variance that corresponds to a point on the frontier. These inputs or variances are the predetermined standards set by the person formulating the portfolio. Stated differently, one seeking the optimum portfolio determines an acceptable input level (indicative of sensitivity) or a given level of variance (indicative of specificity) and selects the genes that lie along the efficient frontier that correspond to that input level or variance. The Wagner Software can select such genes when an input level or variance is selected. It can also assign a weight to each gene in the portfolio as it would for a stock in a stock portfolio.

Determining whether a sample has the condition for which the portfolio is diagnostic can be conducted by comparing the expression of the genes in the portfolio for the patient sample with calculated values of differentially expressed genes used to establish the portfolio. Preferably, a portfolio value is first generated by summing the multiples of the intensity value of each gene in the portfolio by the weight assigned to that gene in the portfolio selection process. A boundary value is then calculated by (Y*standard deviation + mean of the portfolio value for baseline groups) where Y is a stringency value having the same meaning as X described above. A sample having a portfolio value greater than the portfolio value of the baseline class is then classified as having the condition. If desired, this process can be conducted iteratively in accordance with well known statistical methods for improving confidence levels.

Optionally one can reiterate this process until best prediction accuracy is obtained.

The process of portfolio selection and characterization of an unknown is summarized as follows:

1. Choose baseline class
2. Calculate mean, and standard deviation of each gene for baseline class samples
3. Calculate (X*Standard Deviation + Mean) for each gene. This is the baseline reading from which all other samples will be compared. X is a stringency variable with higher values of X being more stringent than lower.
4. Calculate ratio between each Experimental sample versus baseline reading calculated in step 3.
5. Transform ratios such that ratios less than 1 are negative (eg.using Log base 10). (Down regulated genes now correctly have negative values necessary for MV optimization).
6. These transformed ratios are used as inputs in place of the asset returns that are normally used in the software application.
7. The software will plot the efficient frontier and return an optimized portfolio at any point along the efficient frontier.
8. Choose a desired return or variance on the efficient frontier.
9. Calculate the Portfolio's Value for each sample by summing the multiples of each gene's intensity value by the weight generated by the portfolio selection algorithm.
10. Calculate a boundary value by adding the mean Portfolio Value for groups to the multiple of Y and the Standard Deviation of the Baseline's Portfolio Values. Values greater than this boundary value shall be classified as the Experimental Class.
11. Optionally one can reiterate this process until best prediction accuracy is obtained.

Alternatively, genes can first be preselected by identifying those genes whose expression shows some minimal level of differentiation. The pre-selection in this alternative method is preferably based on a threshold given by $1 \leq \left|\frac{(\mu_t - \mu_n)}{(\sigma_t + \sigma_n)}\right|$, where $\mu_t$ is the mean of the subset known to possess the disease or condition, $\mu_n$ is the mean of the subset of normal samples, and $\sigma_t + \sigma_n$ represent the combined standard deviations. A signal to noise cutoff can also be used by pre-selecting the data according to a relationship such as $0.5 \leq \left|\frac{(\mu_t - MAX_n)}{(\sigma_t + \sigma_n)}\right|$. This ensures that genes that are pre-selected based on their differential modulation are differentiated in a clinically significant way. That is, above the noise level of instrumentation appropriate to the task of measuring the diagnostic parameters. For each marker pre-selected according to these criteria, a matrix is established in which columns represents samples, rows represent markers and each element is a normalized intensity measurement for the expression of that marker according to the relationship: $\left|\frac{(\mu_t - I)}{\mu_t}\right|$ where I is the intensity measurement.

It is also possible to set additional boundary conditions to define the optimal portfolios. For example, portfolio size can limited to a fixed range or number of markers. This can be done either by making data pre-selection criteria more stringent (e.g, $.8 \leq \left|\frac{(\mu_t - MAX_n)}{(\sigma_t + \sigma_n)}\right|$ instead of $0.5 \leq \left|\frac{(\mu_t - MAX_n)}{(\sigma_t + \sigma_n)}\right|$) or by using programming features such as restricting portfolio size. One could, for example, set the boundary condition that the efficient frontier is to be selected from among only the most optimal 10 genes. One could also use all of the genes pre-selected for determining the efficient frontier and then limit the number of genes selected (e.g., no more than 10).

The process of selecting a portfolio can also include the application of heuristic rules. Preferably, such rules are formulated based on biology and an understanding of the technology used to produce clinical results. More preferably, they are applied to output from the optimization method. For example, the mean variance method of portfolio selection can be applied to microarray data for a number of genes differentially expressed in subjects with colorectal cancer. Output from the method would be an optimized set of genes that could include some genes that are expressed in peripheral blood as well as in diseased tissue. If samples used in the testing method are obtained from peripheral blood and certain genes differentially expressed in instances of breast cancer could also be differentially expressed in peripheral blood, then a heuristic rule can be applied in which a portfolio is selected from the efficient frontier excluding those that are differentially expressed in peripheral blood. Of course, the rule can be applied prior to the formation of the efficient frontier by, for example, applying the rule during data pre-selection.

Other heuristic rules can be applied that are not necessarily related to the biology in question. For example, one can apply the rule that only a given percentage of the portfolio can be represented by a particular gene or genes. Commercially available software such as the Wagner Software readily accommodates these types of heuristics. This can be useful, for example, when factors other than accuracy and precision (e.g., anticipated licensing fees) have an impact on the desirability of including one or more genes.

One method of the invention involves comparing gene expression profiles for various genes (or portfolios) to ascribe prognoses. The gene expression profiles of each of the genes comprising the portfolio are fixed in a medium such as a computer readable medium. This can take a number of forms. For example, a table can be established into which the range of signals (e.g., intensity measurements) indicative of disease is input. Actual patient data can then be compared to the values in the table to determine whether the patient samples are normal or diseased. In a more sophisticated embodiment, patterns of the expression signals (e.g., flourescent intensity) are recorded digitally or graphically. The gene expression patterns from the gene portfolios used in conjunction with patient samples are then compared to the expression patterns. Pattern comparison software can then be used to determine whether the patient samples have a pattern indicative of recurrence of the disease. Of course, these comparisons can also be used to determine whether the patient is not likely to experience disease recurrence. The expression profiles of the samples are then compared to the portfolio of a control cell. If the sample expression patterns are consistent with the expression pattern for recurrence of a colorectal cancer then (in the absence of countervailing medical considerations) the patient is treated as one would treat a relapse patient. If the sample expression patterns are consistent with the expression pattern from the normal/control cell then the patient is diagnosed negative for colorectal cancer.

Numerous well known methods of pattern recognition are available. The following references provide some examples:

Weighted Voting:

Golub, TR., Slonim, DK., Tamaya, P., Huard, C., Gaasenbeek, M., Mesirov, JP., Coller, H., Loh, L., Downing, JR., Caligiuri, MA., Bloomfield, CD., Lander, ES. Molecular classification of cancer: class discovery and class prediction by gene expression monitoring. Science 286:531-537, 1999

Support Vector Machines:

Su, Al., Welsh, JB., Sapinoso, LM., Kern, SG., Dimitrov, P., Lapp, H., Schultz, PG., Powell, SM., Moskaluk, CA., Frierson, HF. Jr., Hampton, GM. Molecular classification of human carcinomas by use of gene expression signatures. Cancer Research 61:7388-93, 2001

Ramaswamy, S., Tamayo, P., Rifkin, R., Mukherjee, S., Yeang, CH., Angelo, M., Ladd, C., Reich, M., Latulippe, E., Mesirov, JP., Poggio, T., Gerald, W., Loda, M., Lander, ES., Gould, TR. Multiclass cancer diagnosis using tumor gene expression signatures Proceedings of the National Academy of Sciences of the USA 98: 15149-15154, 2001

K-nearest Neighbors:

Ramaswamy, S., Tamayo, P., Rifkin, R., Mukherjee, S., Yeang, CH., Angelo, M., Ladd, C., Reich, M., Latulippe, E., Mesirov, JP., Poggio, T., Gerald, W., Loda, M., Lander, ES., Gould, TR. Multiclass cancer diagnosis using tumor gene expression signatures Proceedings of the National Academy of Sciences of the USA 98:15149-, 15154, 2001

Correlation Coefficients:

van't Veer LJ, Dai H, van de Vijver MJ, He YD, Hart AA, Mao M, Peterse HL, van der Kooy K, Marton MJ, Witteveen AT, Schreiber GJ, Kerkhoven RM, Roberts C, Linsley PS, Bernards R, Friend SH. Gene expression profiling predicts clinical outcome of breast cancer.Nature. 2002 Jan 31;415(6871):530-6.

The gene expression profiles of this invention can also be used in conjunction with other non-genetic diagnostic methods useful in cancer diagnosis, prognosis, or treatment monitoring. For example, in some circumstances it is beneficial to combine the diagnostic power of the gene expression based methods described above with data from conventional markers such as serum protein markers (e.g., carcinoembryonic antigen). A range of such markers exists including such analytes as CEA. In one such method, blood is periodically taken from a treated patient and then subjected to an enzyme immunoassay for one of the serum markers described above. When the concentration of the marker suggests the return of tumors or failure of therapy, a sample source amenable to gene expression analysis is taken. Where a suspicious mass exists, a fine needle aspirate is taken and gene expression profiles of cells taken from the mass are then analyzed as described above. Alternatively, tissue samples may be taken from areas adjacent to the tissue from which a tumor was previously removed. This approach can be particularly useful when other testing produces ambiguous results.

Articles include representations of the gene expression profiles useful for treating, diagnosing, prognosticating, and otherwise assessing diseases. These profile representations are reduced to a medium that can be automatically read by a machine such as computer readable media (magnetic, optical, and the like). The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. A graphical recordation is one such format. Clustering algorithms such as those incorporated in "DISCOVERY" and "INFER" software from Partek, Inc. mentioned above can best assist in the visualization of such data.

Different types of articles of manufacture are media or formatted assays used to reveal gene expression profiles. These can comprise, for example, microarrays in which sequence complements or probes are affixed to a matrix to which the sequences indicative of the genes of interest combine creating a readable determinant of their presence. Alternatively, articles can be fashioned into reagent kits for conducting hybridization, amplification, and signal generation indicative of the level of expression of the genes of interest for detecting colorectal cancer.

Kits made according to the invention include formatted assays for determining the gene expression profiles. These can include all or some of the materials needed to conduct the assays such as reagents and instructions.

The invention is further illustrated by the following non-limiting examples.

**Examples:** Genes analyzed according to this invention are typically related to full-length nucleic acid sequences that code for the production of a protein or peptide. One skilled in the art will recognize that identification of full-length sequences is not necessary from an analytical point of view. That is, portions of the sequences or ESTs can be selected according to well-known principles for which probes can be designed to assess gene expression for the corresponding gene.

**Example 1- Sample Handling and LCM.**

Fresh frozen tissue samples were collected from patients who had surgery for colorectal tumors. The samples that were used were from 63 patients staged with Duke's B according to standard clinical diagnostics and pathology. Clinical outcome of the patients was known. Thirty-six of the patients have remained disease-free for more than 3 years while 27 patients had tumor relapse within 3 years.

The tissues were snap frozen in liquid nitrogen within 20-30 minutes of harvesting, and stored at -80C° thereafter. For laser capture, the samples were cut (6μm), and one section was mounted on a glass slide, and the on film (P.A.L.M.), which had been fixed onto a glass slide (Micro Slides Colorfrost, VWR Scientific, Media, PA). The section mounted on a glass slide was after fixed in cold acetone, and stained with Mayer's Haematoxylin (Sigma, St. Louis, MO). A pathologist analyzed the samples for diagnosis and grade. The clinical stage was estimated from the accompanying surgical pathology and clinical reports to verify the Dukes classification. The section mounted on film was after fixed for five minutes in 100% ethanol, counter stained for 1 minute in eosin/100% ethanol (100μg of Eosin in 100ml of dehydrated ethanol), quickly soaked once in 100% ethanol to remove the free stain, and air dried for 10 minutes.

Before use in LCM, the membrane (LPC-MEMBRANE PEN FOIL 1.35 μm No 8100, P.A.L.M. GmbH Mikrolaser Technologie, Bernried, Germany) and sides were pretreated to abolish RNases, and to enhance the attachment of the tissue sample onto the film. Briefly, the slides were washed in DEP $H_2O$, and the film was washed in RNase AWAY (Molecular Bioproducts, Inc., San Diego, CA) and rinsed in DEP $H_2O$. After attaching the film onto the glass slides, the were baked at +120°C for 8 hours, treated with TI-SAD (Diagnostic Products Corporation, Los Angeles, CA, 1:50 in DEP $H_2O$, filtered through cotton wool), and incubated at for 30 minutes. Immediately before use, a 10μl aliquot of RNase inhibitor solution (Rnasin Inhibitor 2500U=33U/μl N211A, Promega GmbH, Mannheim, Germany, 0.5μl in 400μl of freezing solution, containing 0.15 mol NaCl, 10 mmol Tris pH 8.0, 0.25 mmol dithiothreitol) was spread onto the film, where the tissue sample was to be mounted.

The tissue sections mounted on film were used for LCM. Approximately 2000 epithelial cells/sample were captured using the PALM Robot-Microbeam technology (P.A.L.M. Mikrolaser Technologie, Carl Zeiss, Inc., Thornwood, NY), coupled into Zeiss Axiovert 135 microscope (Carl Zeiss Jena GmbH, Jena, Germany). The surrounding stroma in the normal mucosa, and the occasional intervening stromal components in cancer samples, were included. The captured cells were put in tubes in 100% ethanol and preserved at -80°C.

**Example 2- RNA Extraction and Amplification.**

Zymo-Spin Column (Zymo Research, Orange, CA 92867) was used to extract total RNA from the LCM captured samples. About 2 ng of total RNA was resuspended in 10 ul of water and 2 rounds of the T7 RNA polymerase based amplification were performed to yield about 50 ug of amplified RNA.

**Example 3- cDNA Microarray Hybridization and Quantitation.**

A set of cDNA microarrays consisting of approximately 23,000 human cDNA clones was used to test the samples by use of the humanU133a chip obtained and commercially available from Affymetrix, Inc. Total obtained and prepared as outlined above and applied to the chips and analyzed by Agilent BioAnalyzer according to the manufacturer's protocol. All 63 samples passed the quality control standards and the data were used for marker selection.

Chip intensity data was analyzed using MAS Version 5.0 software commercially available from Affymetrix, Inc. ("MAS 5.0"). An unsupervised analysis was used to identify two genes that distinguish patients that would relapse from those who would not as follows. The chip intensity data obtained as described was the input for the unsupervised clustering software commercially available as PARTEK version 5.1 software. This unsupervised clustering algorithm identified a group of 20 patients with a high frequency of relapse (13 relapsers and 7 survivors). From the original 23,000 genes, t-testing analysis selected 276 genes that significantly differentially expressed in these patients. From this group, two genes were selected that best distinguish relapsing patients from those that do not relapse: Human intestinal peptide-associated transporter (Seq. ID. No. 3) and Homo sapiens fatty acid binding protein 1 (Seq. ID No. 1). These two genes are down-regulated (in fact, they are turned off or not expressed) in the relapsing patients from this patient group. This is shown in Figure 1 and Figure 2 in which signal intensity is plotted (y-axis) against patient sample number (x-axis).

Supervised analysis was then conducted to further discriminate relapsing patients from those who did not relapse in the remaining 43 patients. This group of patient data was then divided into the following groups: 27 patients were assigned as the training set and 16 patients were assigned as the testing set. This ensured that the same data was not used to both identify markers and then validate their utility.

An unequal variance t-test was performed on the training set. From a list of 28 genes that have significant corrected p values, MHC II-DR-B was chosen. These genes are down-regulated in relapsers. MHC II-DR-B (Seq. ID No. 2) also had the smallest p-value (Figure 3a).

In an additional round of supervised analysis, a variable selection procedure for linear discriminant analysis was implemented using the Partek Version 5.0 software described above to separate relapsers from survivors in the training set. The search method was forward selection. The variable selected with the lowest posterior error was immunoglobulin-like transcript 5 protein (Seq. ID No. 4) (Figure 3b). A Cox proportional hazard model (using "S Plus" software from Insightful, Inc.) was then used for gene selection to confirm gene selection identified above for survival time. In each cycle of total 27 cycles, each of the 27 patients in the training set was held out, the remaining 26 patients were used in the univariate Cox model regression to assess the strength of association of gene expression with the patient survival time. The strength of such association was evaluated by the corresponding estimated standardized parameter estimate and P value returned from the Cox model regression. P value of 0.01 was used as the threshold to select top genes from each cycle of the leave-one-out gene selection. The top genes selected from each cycle were then compared in order to select those genes that showed up in at least 26 times in the total of 27 leave-one-out gene selection cycles. A total of 70 genes were selected and both MHC II-DR-B and immunoglobulin-like transcript 5 protein were among them (Again, showing down regulation).

***Construction of a multiple-gene predictor:*** Two genes, MHC II-DR-B and immunoglobulin-like transcript 5 protein were used to produce a predictor using linear discriminant analysis. The voting score was defined as the posterior probability of relapse. If the patient score was greater than 0.5, the patient was classified as a relapser. If the patient score was less than 0.5, the patient was classified as a survivor. The predictor was tested on the training set (Table 1). The Kaplan -Meier curve was constructed on the predicted relapsers and survivors (Figure 4)

***Cross-validation and evaluation of predictor:*** Performance of the predictor should be determined on an independent data set because most classification method work well on the examples that were used in their establishment. The 16 patients test set used to assess prediction accuracy. The cutoff for the classification was determined by using the ROC curve (Figure 5). With the selected cutoff, the numbers of correct prediction for relapse and survival patients in the test set were determined and are summarized in (Table 2). The Kaplan -Meier curve was constructed on the predicted relapsers and survivors (Figure 6). ***Overall prediction:*** Gene expression profiling of 63 Duke's B colon cancer patients led to identification of 4 genes that have differential expression (down regulation or turned off) in these patients. These genes are Seq. ID No. 1, Seq. ID No. 2, Seq. ID No. 3, and Seq. ID No. 4. Thirty-six of the patients have remained disease-free for more than years while 27 patients had tumor relapse within 3 years. Using the 3 gene markers portfolio of Seq. ID No. 2, Seq. ID No. 3, and Seq. ID No. 4, 22 of the 27 relapse patients and 27 of 36 disease-free patients are identified correctly. This result represents a sensitivity of 82% and a specificity of 75%. The positive predictive value is 71% and the negative predictive value is 84% (Table 3). The Kaplan -Meier curve was constructed on the predicted relapsers and survivors (Figure 6).

The genes comprising the profiles of this invention are described below.

**Homo sapiens fatty acid binding protein 1 (FABP1):**

Human liver fatty acid binding protein (L-FABP) gene was first identified in a liver cDNA library by Smith LC et. al. in J. Biol. Chem. 260 (5), 2629-2632 (1985). The L-FABP contains 127 amino acid residues. Fatty acid binding proteins are a family of small, highly conserved, cytoplasmic proteins that bind long-chain fatty acids and other hydrophobic ligands. It is thought that FABPs roles include fatty acid uptake, transport, and metabolism. They may also be responsible in the modulation of cell growth and proliferation. L-FABP shares significant homology with I-FABP which is specifically expressed in colon tissue.,

**Human intestinal peptide-associated transporter HPT-1 mRNA:**

This gene was identified by a group of scientist from Eli Lilly and Company. The paper was published in Science 1994 Apr 15;264(3137):430-3. This gene encodes an approximately 92-kilodalton membrane protein, and the amino acid sequence indicated that this transport-associated protein shares several conserved structural elements with the cadherin superfamily of calcium-dependent, cell-cell adhesion proteins.

**Homo sapiens MHC class II antigen (HLA-DRB1) mRNA:**

This gene was found first from a Spanish intent in 1997, and published in Tissue Antigens 1997 Jun;49(6):

658-61. As its name indicated that it belongs to the super family of MHC class II antigens. This gene encodes a protein product of 267 amino acids.

**Homo sapiens clone 6 immunoglobulin-like transcript 5 protein mRNA:**

This gene encodes a protein product that is a inhibitory MHC class I receptor of the immunoglobulin-superfamily, expressed not only by subsets of NK and T cells, but also by B cells, monocytes, macrophages, and dendritic cells. This molecule contains 194 amino acids. The sequence was published in J Exp Med 1997 Dec 1;186(11):1809-18. This receptor binds MHC class I molecules and delivers a negative signal that inhibits killing by NK and T cells, as well as Ca2+ mobilization in B cells and myelomonocytic cells triggered through the B cell antigen receptor and human histocompatibility leukocyte antigens (HLA)-DR, respectively.

**Homo sapiens hydroxymethylbilane synthase (also called Porphobilinogen deaminase-PBGD):**

This gene was used as the control gene. It is one of the least variable genes between solid tumor and normal tissues. The sequence was first published in Nucleic Acids Res. 14 (15), 5955-5968 (1986).

**Table 1. Prediction accuracy od training set using 2-gene predictor.**

| Study | Number of Sample | Correct Prediction |
|---|---|---|
| Relapse | 6 | 5 |
| Survivor | 21 | 21 |
| Sensitivity | 83% | |
| Specificity | 100% | |

**Table 2. Prediction accuracy based on testing set using 2-gene predictor.**

| Study | Number of Sample | Correct Prediction |
|---|---|---|
| Relapse | 8 | 4 |
| Survivor | 8 | 7 |
| Sensitivity | 50% | |
| Specifiticity | 88% | |

**Table 3. Prediction accuracy based on all patients using 3-gene predictor (Seq. ID 2, Seq. ID 3, and Seq. ID 4).**

| Study | Number of Sample | Correct Prediction |
|---|---|---|
| Relapse | 27 | 22 |
| Survivor | 36 | 28 |
| Sensitivity | 82% | |
| Specificity | 75% | |

## Claims

1. A method of assessing colorectal cancer status comprising identifying differential modulation of each gene (relative to the expression of the same genes in a normal population) in a combination of genes consisting of Seq. ID. No. 2, Seq. ID. No. 3 and Seq. ID. No. 4.

2. The method of claim 1 or claim 2 wherein the combination of genes further includes Seq. ID. No. 1.

3. The method of claim 1 or claim 2 wherein there is at least a 2 fold difference in the expression of the modulated genes.

4. The method of claim 1 or claim 2 wherein the p-value indicating differential modulation is less than 0.05.

5. The method of claim 1or claim 2 further comprising a colorectal diagnostic that is not genetically based.

6. A kit comprising isolated nucleic acid sequences, their complements, or portions thereof of a combination of genes consisting of Seq. ID. No. 2, Seq. ID. No. 3, and Seq. ID. No. 4.

7. The kit of claim 6 wherein the combination of genes further includes Seq. ID. No. 1.

8. The kit of claim 6 or claim 7 wherein said nucleic acid sequences, their complements, or portions thereof are in a matrix suitable for identifying the differential expression of the genes contained therein.

9. The kit of claim 8 wherein said matrix is employed in a microarray.

10. The kit of claim 9 wherein said microarray is a cDNA microarray.

11. The kit of claim 9 wherein said microarray is an oligonucleotide microarray.

12. The kit of claim 6 or claim 7 comprising reagents for conducting a microarray analysis.

13. The kit of claim 6 or claim 7 further comprising a medium through which said nucleic acid sequences, their compliments, or portions thereof are assayed.

14. A method of assessing response to treatment for colorectal cancer comprising identifying differential modulation of each gene (relative to the expression of the same genes in a normal population) in a combination of genes consisting of Seq. ID. No. 2, Seq. ID. No. 3, and Seq. ID. No. 4.

15. The method of claim 14, wherein the combination of genes further includes Seq. ID. No. 1.

## Patentansprüche

1. Verfahren zum Beurteilen von kolorektralem Krebsstatus, umfassend Identifizieren einer unterschiedlichen Modulation von jedem Gen (relativ zu der Expression der gleichen Gene in einer normalen Population) in einer Genkombination, bestehend aus SEQ ID Nr: 2, SEQ ID Nr: 3 und SEQ ID Nr:4.

**2.** Verfahren nach Anspruch 1, wobei die Genkombination ferner SEQ ID Nr: 1 enthält.

**3.** Verfahren nach Anspruch 1 oder Anspruch 2, wobei ein mindestens 2-facher Unterschied in der Expression der modulierten Genen besteht.

**4.** Verfahren nach Anspruch 1 oder Anspruch 2, wobei der p-Wert, der eine unterschiedliche Modulation anzeigt, kleiner als 0,05 ist.

**5.** Verfahren nach Anspruch 1 oder Anspruch 2, weiter umfassend eine kolorektale Diagnose, die nicht genetisch ist.

**6.** Kit, umfassend isolierte Nukleinsäuresequenzen, ihre Komplentäre, oder Teile davon aus einer Genkombination, bestehend aus SEQ ID Nr: 2, SEQ ID Nr: 3 und SEQ ID Nr: 4.

**7.** Kit nach Anspruch 6, wobei die Genkombination ferner SEQ ID Nr: 1 enthält.

**8.** Kit nach Anspruch 6 oder Anspruch 7, wobei die Nukleinsäuresequenzen, ihre Komplentäre, oder Teile davon in einer Matrix sind, die zum Identifizieren der unterschiedlichen Expression der darin enthaltenen Genen geeignet ist.

**9.** Kit nach Anspruch 8, wobei die Matrix in einem Mikroarray eingesetzt wird.

**10.** Kit nach Anspruch 9, wobei das Mikroarray ein cDNA-Mikroarray ist.

**11.** Kit nach Anspruch 10, wobei das Mikroarray ein Oligonukleotid-Mikroarray ist.

**12.** Kit nach Anspruch 6 oder Anspruch 7, umfassend Reagenzien zum Durchführen einer Mikroarray-Analyse.

**13.** Kit nach Anspruch 6 oder Anspruch 7, weiter umfassend ein Medium, mit dem die Nukleinsäuresequenzen, ihre Komplementäre, oder Teile davon untersucht werden.

**14.** Verfahren zum Beurteilen des Ansprechens einer Behandlung von kolorektalem Krebs, umfassend Identifizieren einer unterschiedlichen Modulation von jedem Gen (relativ zu der Expression der gleichen Gene in einer normalen Population) in einer Genkombination bestehend aus SEQ ID Nr: 2, SEQ ID Nr: 3 und SEQ ID Nr:4.

**15.** Verfahren nach Anspruch 14, wobei die Genkombination ferner SEQ ID Nr: 1 beinhaltet.

## Revendications

**1.** Procédé d'évaluation de l'évolution du cancer colorectal comprenant l'identification de la modulation différentielle de chaque gène (par rapport à l'expression des mêmes gènes dans une population saine) dans une combinaison de gènes consistant en SEQ. ID. No. 2, SEQ. ID. No. 3 et SEQ. ID. No. 4.

**2.** Procédé selon la revendication 1 ou 2, dans lequel la combinaison de gènes comprend en outre la SEQ. ID. No. 1.

**3.** Procédé selon la revendication 1 ou 2, dans lequel il y a au moins une différence de deux fois dans l'expression des gènes modulés.

**4.** Procédé selon la revendication 1 ou 2, dans lequel la valeur p indiquant la modulation différentielle est inférieure à 0,05.

**5.** Procédé selon la revendication 1 ou 2, comprenant en outre un diagnostic colorectal n'étant pas basé sur une étude génétique.

**6.** Kit comprenant des séquences d'acides nucléiques isolées, leurs compléments, ou parties de celles-ci d'une combinaison de gènes consistant en SEQ. ID. No. 2, SEQ. ID. No. 3 et SEQ. ID. No. 4.

7. Kit selon la revendication 6, dans lequel la combinaison de gènes comprend en outre la SEQ. ID. No. 1.

8. Kit selon la revendication 6 ou 7, dans lequel lesdites séquences d'acides nucléiques, leurs compléments, ou parties de celles-ci sont dans une matrice adaptée à l'identification de l'expression différentielle des gènes contenus dans celle-ci.

9. Kit selon la revendication 8, dans lequel ladite matrice est employée dans une puce.

10. Kit selon la revendication 9, dans lequel ladite puce est une puce à ADNc.

11. Kit selon la revendication 9, dans lequel ladite puce est une puce à oligonucléotides.

12. Kit selon la revendication 6 ou 7, comprenant des réactifs pour réaliser une analyse de puce.

13. Kit selon la revendication 6 ou 7, comprenant en outre un milieu dans lequel lesdites séquences d'acides nucléiques, leurs compléments, ou parties de celles-ci sont dosés.

14. Procédé d'évaluation d'une réponse à un traitement pour le cancer colorectal comprenant l'identification de la modulation différentielle de chaque gène (par rapport à l'expression des mêmes gènes dans une population normale) dans une combinaison de gènes consistant en SEQ. ID. No. 2, SEQ. ID. No. 3 et SEQ. ID. No. 4.

15. Procédé selon la revendication 14, dans lequel la combinaison de gènes comprend en outre la SEQ. ID. No. 1.

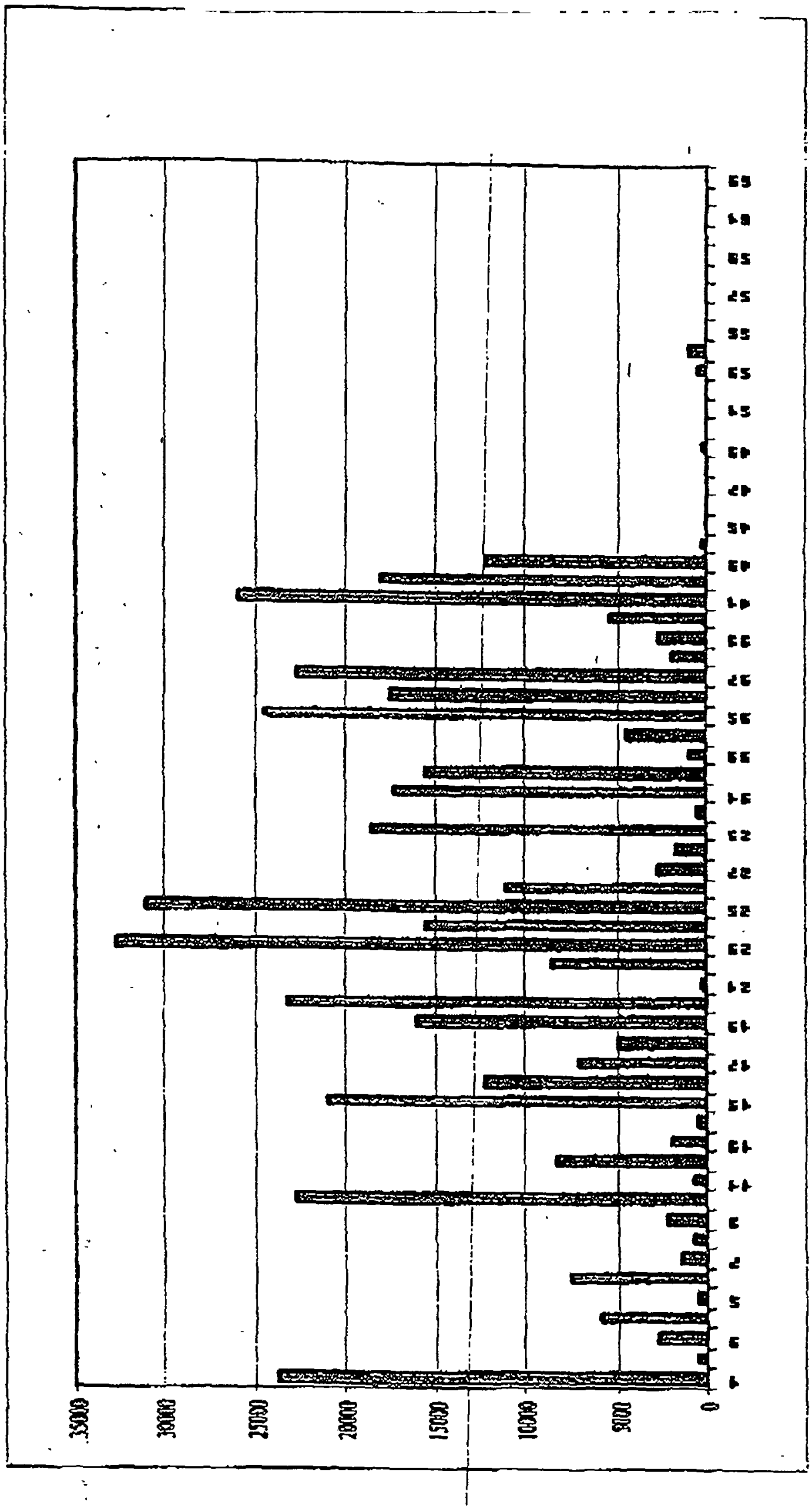


Figure 1

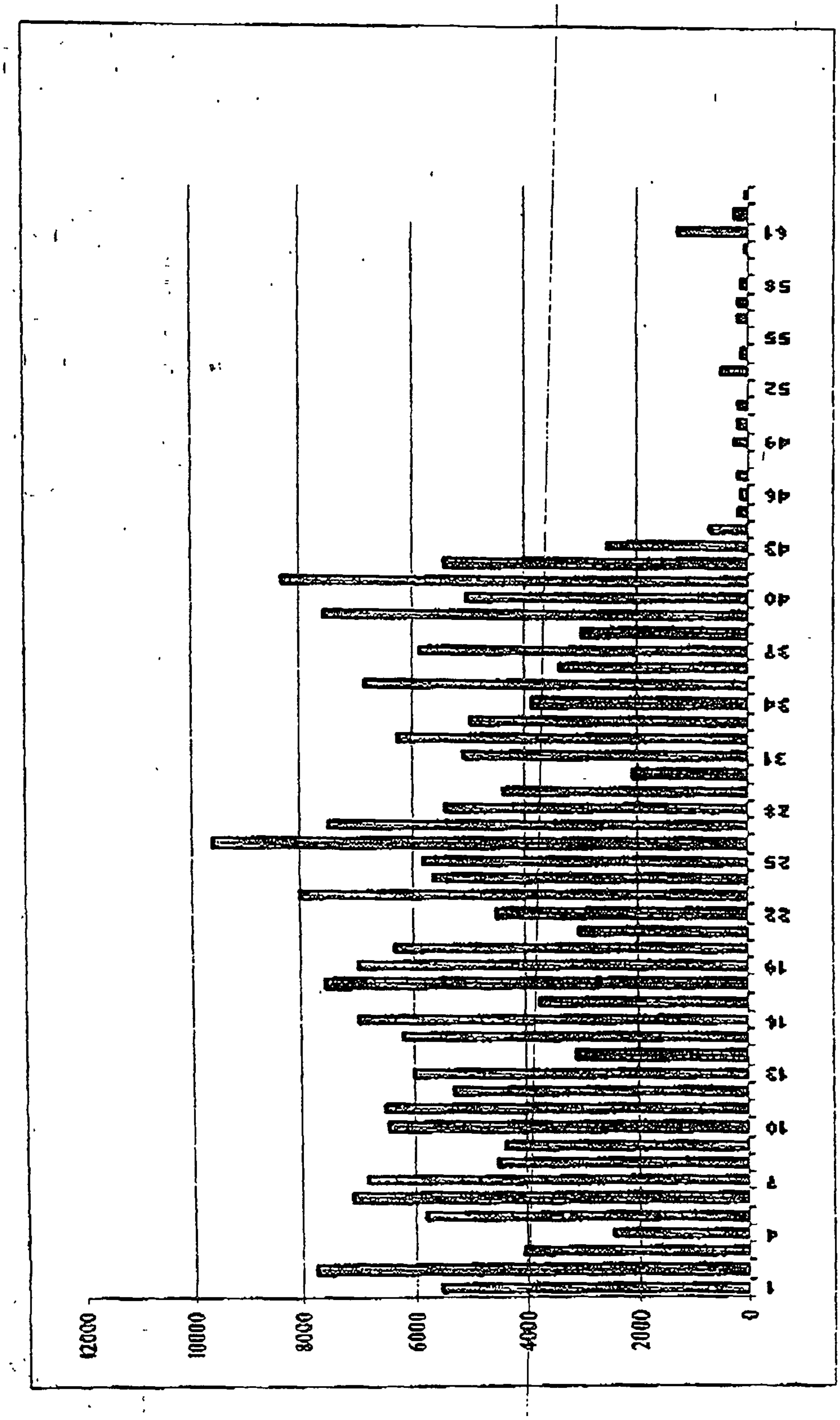
12000
10000
8000
6000
4000
2000
0
1
4
7
10
13
16
19
22
25
28
31
34
37
40
43
46
49
52
55
58
61

Figure 2

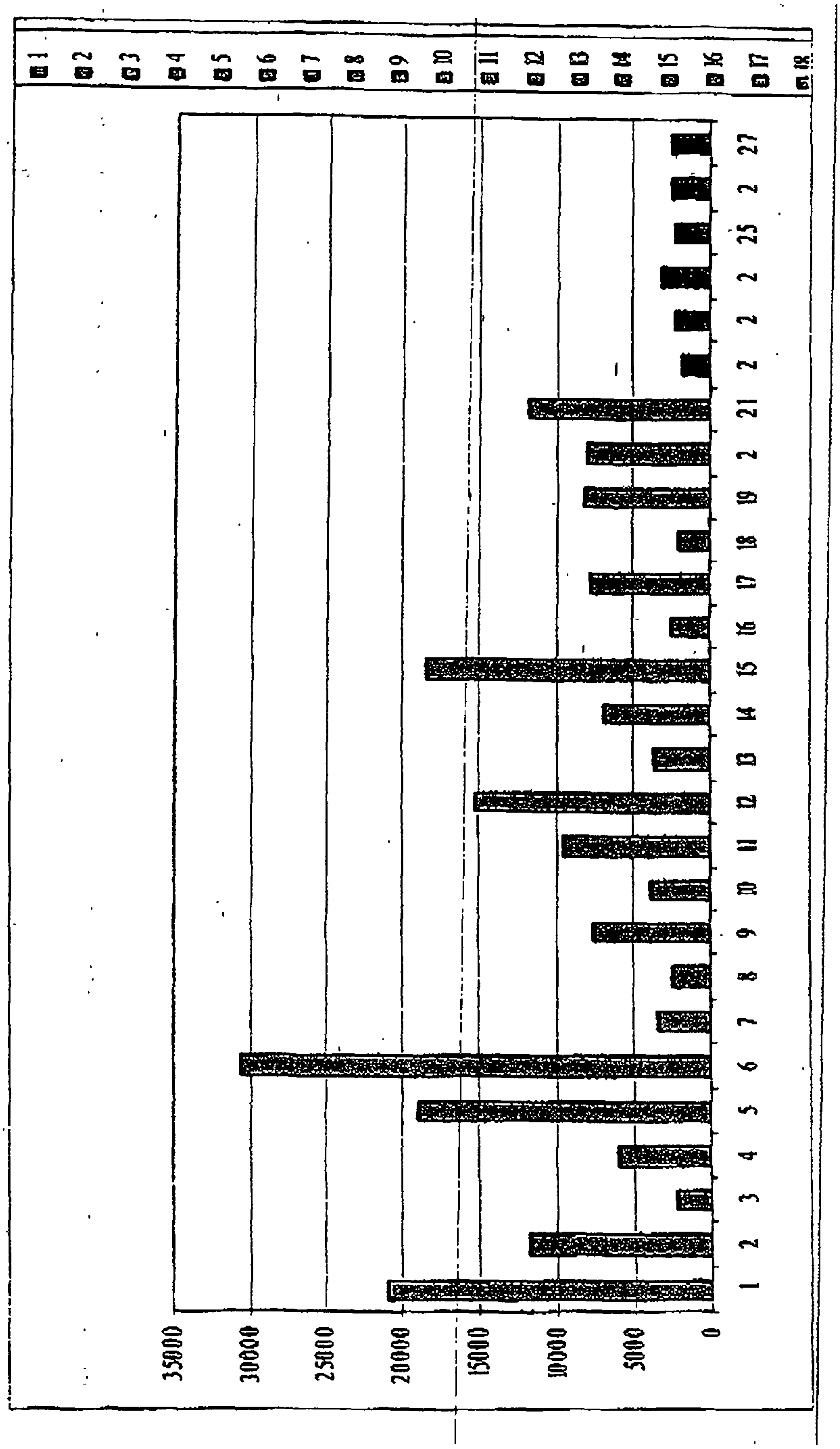


Figure 3A

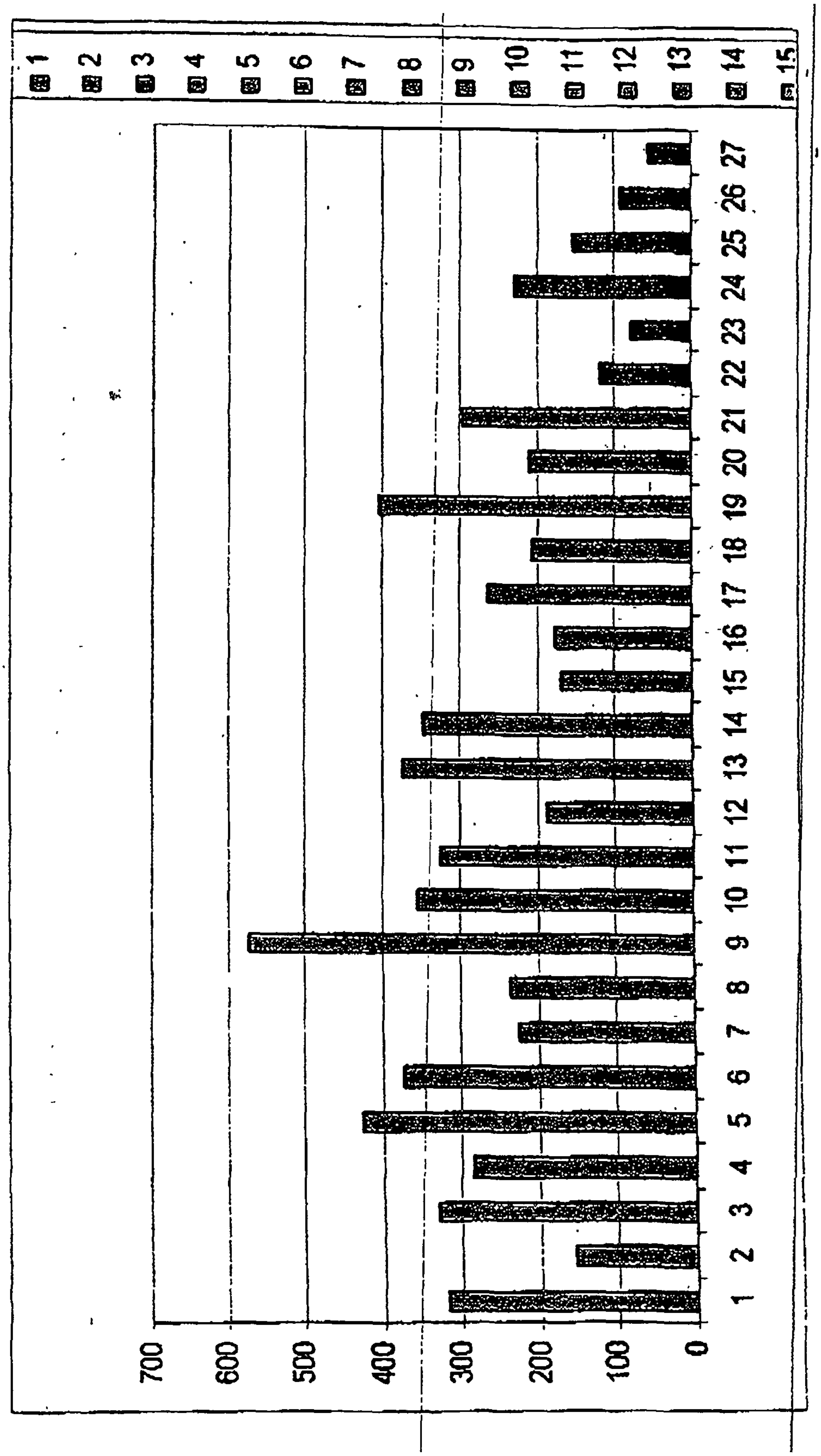
1
2
3
4
5
6
7
8
9
10
11
12
13
14
15
700
600
500
400
300
200
100
0
1 2 3 4 5 6 7 8 9 10 11 12 13 14 15 16 17 18 19 20 21 22 23 24 25 26 27

Figure 3B

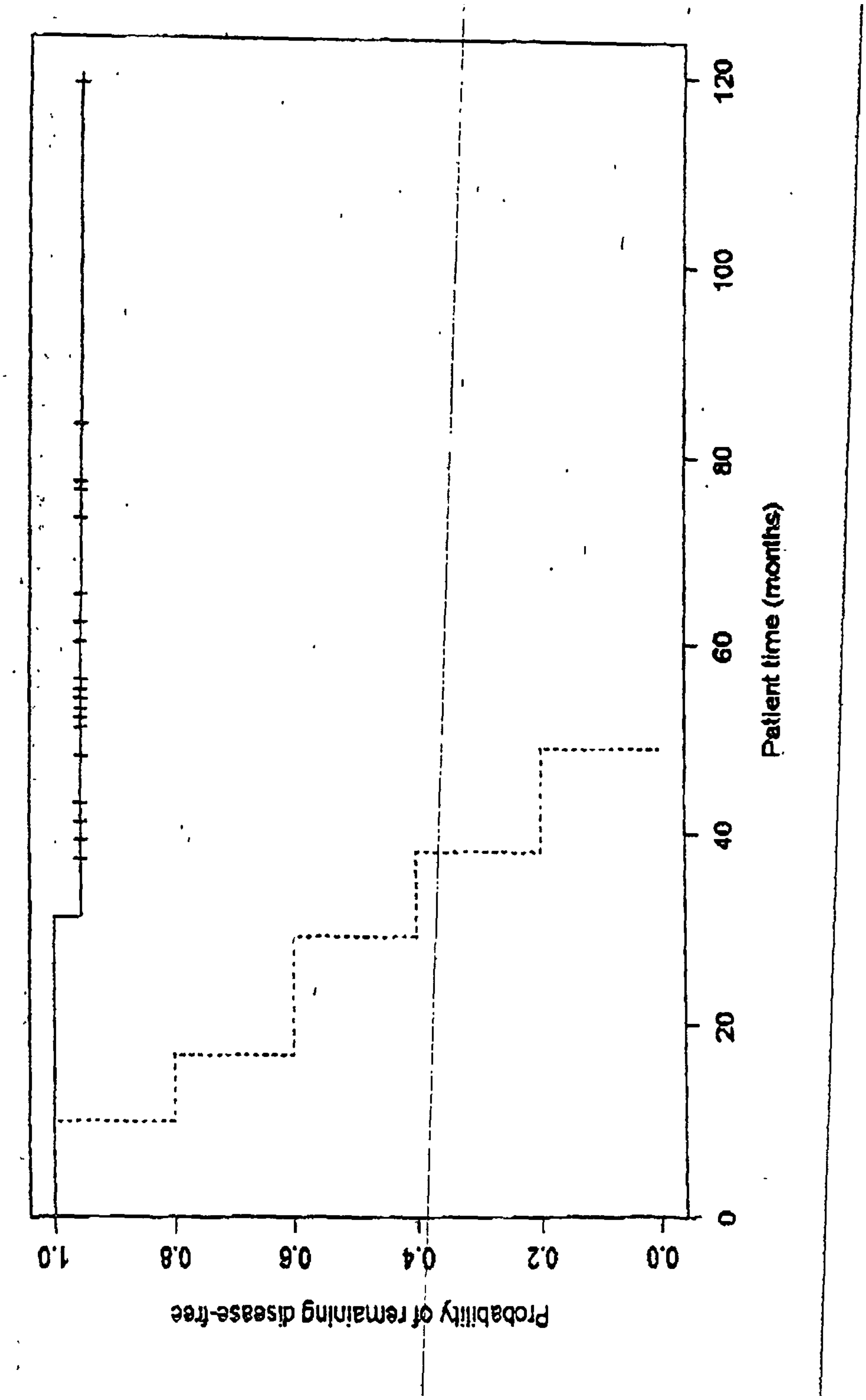
Probability of remaining disease-free
1.0
0.8
0.6
0.4
0.2
0.0
0
20
40
60
80
100
120
Patient time (months)

Figure 4

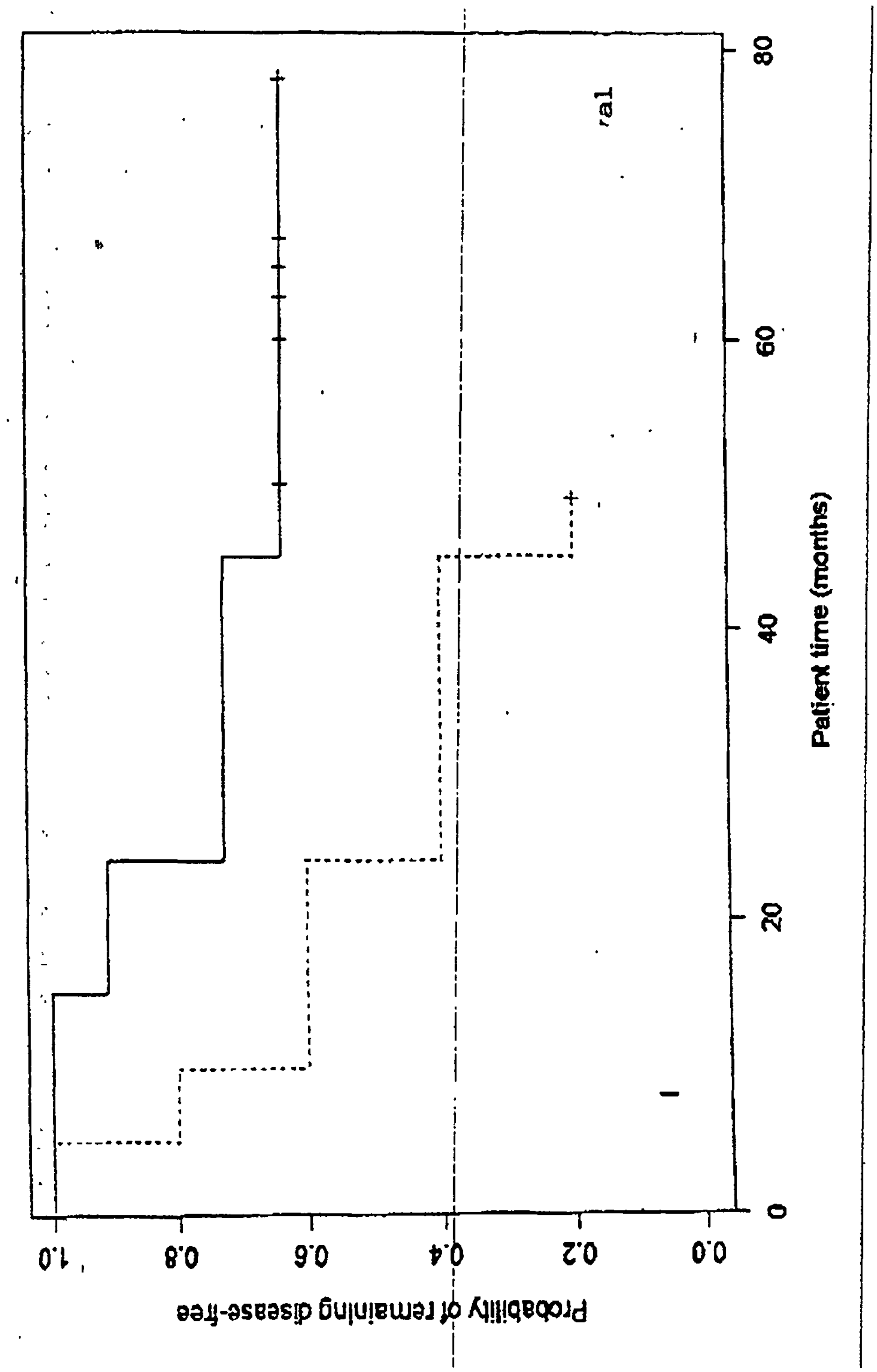
Probability of remaining disease-free
1.0
0.8
0.6
0.4
0.2
0.0
0
20
40
60
80
Patient time (months)

Figure 5.

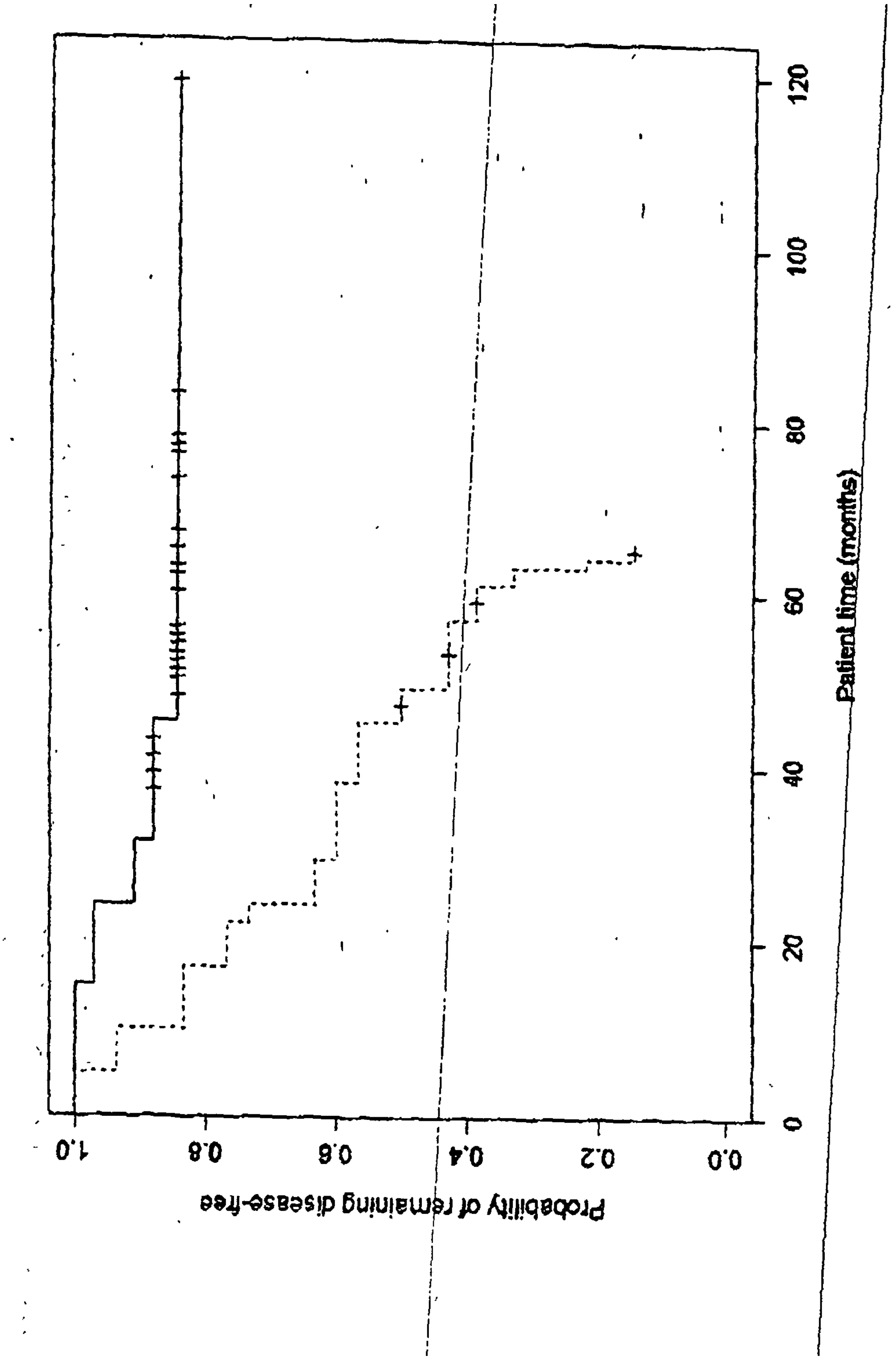
Probability of remaining disease-free
1.0
0.8
0.6
0.4
0.2
0.0
0
20
40
60
80
100
120
Patient time (months)

Figure 6

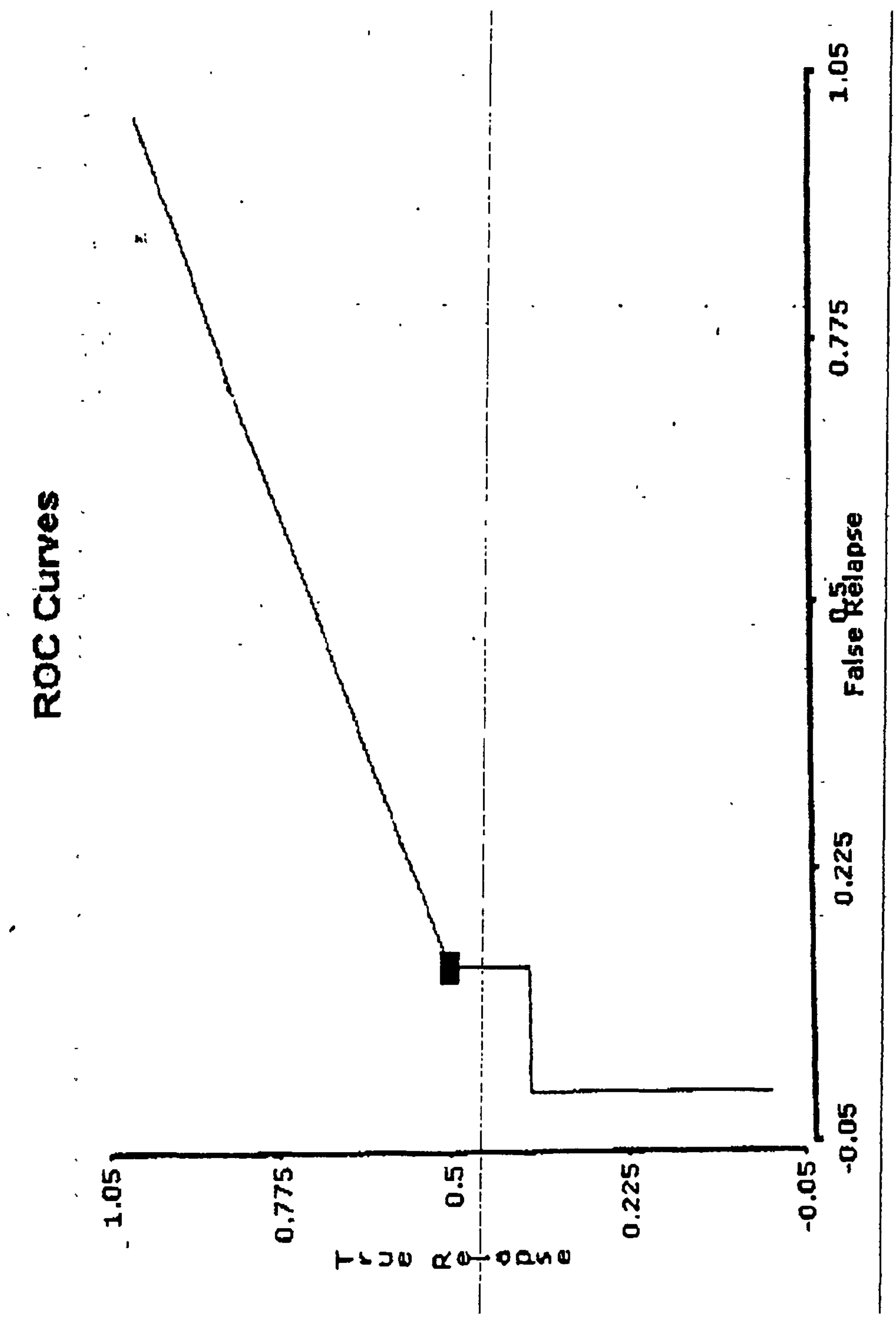
ROC Curves
1.05
0.775
0.5
0.225
-0.05
True Relapse
-0.05
0.225
0.5
0.775
1.05
False Relapse

Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0019206 A
- WO 0194629 A
- US 6136182 A
- US 6271002 B, Linsley
- US 6218122 B, Friend
- US 6218114 B, Peck
- US 6004755 B, Wang


### Non-patent literature cited in the description


- **GOLUB, TR. ; SLONIM, DK. ; TAMAYA, P. ; HUARD, C. ; GAASENBEEK, M. ; MESIROV, JP. ; COLLER, H. ; LOH, L. ; DOWNING, JR. ; CALIGIURI, MA.** Molecular classification of cancer: class discovery and class prediction by gene expression monitoring. *Science,* 1999, vol. 286, 531-537
- **SU, AI. ; WELSH, JB. ; SAPINOSO, LM. ; KERN, SG. ; DIMITROV, P. ; LAPP, H. ; SCHULTZ, PG. ; POWELL, SM. ; MOSKALUK, CA. ; FRIERSON, HF. JR.** Molecular classification of human carcinomas by use of gene expression signatures. *Cancer Research,* 2001, vol. 61, 7388-93
- **RAMASWAMY, S. ; TAMAYO, P. ; RIFKIN, R. ; MUKHERJEE, S. ; YEANG, CH. ; ANGELO, M. ; LADD, C. ; REICH, M. ; LATULIPPE, E. ; MESIROV, JP.** Multiclass cancer diagnosis using tumor gene expression signatures. *Proceedings of the National Academy of Sciences of the USA,* 2001, vol. 98, 15149-15154
- **RAMASWAMY, S. ; TAMAYO, P. ; RIFKIN, R. ; MUKHERJEE, S. ; YEANG, CH. ; ANGELO, M. ; LADD, C. ; REICH, M. ; LATULIPPE, E. ; MESIROV, JP.** Multiclass cancer diagnosis using tumor gene expression signatures. *Proceedings of the National Academy of Sciences of the USA,* 2001, vol. 98, 15149, 15154
- **VAN'T VEER LJ ; DAI H ; VIJVER MJ ; HE YD ; HART AA ; MAO M ; PETERSE HL ; VAN DER KOOY K ; MARTON MJ ; WITTEVEEN AT.** Gene expression profiling predicts clinical outcome of breast cancer. *Nature,* 31 January 2002, vol. 415 (6871), 530-6
- **SMITH LC.** *J. Biol. Chem.,* 1985, vol. 260 (5), 2629-2632
- *Science,* 15 April 1994, vol. 264 (3137), 430-3
- *Tissue Antigens,* June 1997, vol. 49 (6), 658-61
- *J Exp Med,* 01 December 1997, vol. 186 (11), 1809-18
- *Nucleic Acids Res.,* 1986, vol. 14 (15), 5955-5968

SEQUENCE LISTING

<110> Wang, Yixin

<120> Colorectal Cancer Prognostics

<130> CD550005

<140> tbd
<141> 2003-03-31

<160> 5

<170> PatentIn version 3.1

<210> 1
<211> 489
<212> DNA
<213> human

<400> 1

```
agagccgcag gtcagtcgtg aagagggagc tctattgcca ccatgagttt ctccggcaag      60
taccaactgc agagccagga aaactttgaa gccttcatga aggcaatcgg tctgccggaa     120
gagctcatcc agaaggggaa ggatatcaag gggtgtcgg aaatcgtgca gaatgggaag      180
cacttcaagt tcaccatcac cgctgggtcc aaagtgatcc aaaacgaatt cacggtgggg     240
gaggaatgtg agctggagac aatgacaggg gagaaagtca agacagtggt tcagttggaa     300
ggtgacaata aactggtgac aactttcaaa aacatcaagt ctgtgaccga actcaacggc     360
gacataatca ccaataccat gacattgggt gacattgtct tcaagagaat cagcaagaga     420
atttaaacaa gtctgcattt catattattt tagtgtgtaa aattaatgta ataaagtgaa     480
ctttgtttt                                                             489
```

<210> 2
<211> 853
<212> DNA
<213> human

<400> 2

```
gcctgctgct ctggcccctg gtcctgtcct gttctccagc atggtgtgtc tgaggctccc      60
tggaggctcc tgcatggcag ttctgacagt gacactgatg gtgctgagct ccccactggc     120
tttggctggg gacaccagac cacgtttctt ggagtactct acgtctgagt gtcatttctt     180
caatgggacg gagcgggtgc ggtacctgga cagatacttc cataaccagg aggagaacgt     240
gcgcttcgac agcgacgtgg gggagttccg ggcggtgacg gagctggggc ggcctgctgc     300
ggagcactgg aacagccaga aggacctcct ggagcagaag cggggccggg tggacaacta     360
ctgcagacac aactacgggg ttgtggagag cttcacagtg cagcggcgag tccatcctaa     420
ggtgactgtg tatccttcaa agacccagcc cctgcagcac cataacctcc tggtctgttc     480
tgtgagtggt ttctatccag gcagcattga agtcaggtgg ttccggaatg gccaggaaga     540
```

gaagactggg gtggtgtcca caggcctgat ccacaatgga gactggacct tccagaccct 600
ggtgatgctg gaaacagttc ctcggagtgg agaggtttac acctgccaag tggagcaccc 660
aagcgtgaca agccctctca cagtggaatg gagagcacgg tctgaatctg cacagagcaa 720
gatgctgagt ggagtcgggg gctttgtgct gggcctgctc ttccttgggg ccgggctgtt 780
catctacttc aggaatcaga aaggacactc tggacttcag ccaagaggat tcctgagctg 840
aagtgcagat gac 853

<210> 3
<211> 3345
<212> DNA
<213> human

<400> 3

gaattccgtc tcgaccactg aatggaagaa aaggactttt aaccaccatt ttgtgactta 60
cagaaaggaa tttgaataaa gaaaactatg atacttcagg cccatcttca ctccctgtgt 120
cttcttatgc tttatttggc aactggatat ggccaagagg ggaagtttag tggacccctg 180
aaacccatga catttctat ttatgaaggc caagaaccga gtcaaattat attccagttt 240
aaggccaatc ctcctgctgt gacttttgaa ctaactgggg agacagacaa catatttgtg 300
atagaacggg agggacttct gtattacaac agagccttgg acagggaaac aagatctact 360
cacaatctcc aggttgcagc cctggacgct aatggaatta tagtggaggg tccagtccct 420
atcaccatag aagtgaagga catcaacgac aatcgaccca cgtttctcca gtcaaagtac 480
gaaggctcag taaggcagaa ctctcgccca ggaaagccct tcttgtatgt caatgccaca 540
gacctggatg atccggccac tcccaatggc cagcttttatt accagattgt catccagctt 600
cccatgatca acaatgtcat gtactttcag atcaacaaca aaacgggagc catctctctt 660
acccgagagg gatctcagga attgaatcct gctaagaatc cttcctataa tctggtgatc 720
tcagtgaagg acatgggagg ccagagtgag aattccttca gtgataccac atctgtggat 780
atcatagtga cagagaatat ttggaaagca ccaaaacctg tggagatggt ggaaaactca 840
actgatcctc accccatcaa aatcactcag gtgcggtgga atgatcccgg tgcacaatat 900
tccttagttg acaaagagaa gctgccaaga ttcccattt caattgacca ggaaggagat 960
atttacgtga ctcagccctt ggaccgagaa gaaaaggatg catatgtttt ttatgcagtt 1020
gcaaaggatg agtacggaaa accactttca tatcgctgg aaattcatgt aaaagttaaa 1080
gatattaatg ataatccacc tacatgtccg tcaccagtaa ccgtatttga ggtccaggag 1140
aatgaacgac tgggtaacag tatcgggacc cttactgcac atgacaggga tgaagaaaat 1200
actgccaaca gttttctaaa ctacaggatt gtggagcaaa ctcccaaact tcccatggat 1260
ggactcttcc taatccaaac ctatgctgga atgttacagt tagctaaaca gtccttgaag 1320

aagcaagata ctcctcagta caacttaacg atagaggtgt ctgacaaaga tttcaagacc 1380
ctttgttttg tgcaaatcaa cgttattgat atcaatgatc agatccccat ctttgaaaaa 1440
tcagattatg gaaacctgac tcttgctgaa gacacaaaca ttgggtccac catcttaacc 1500
atccaggcca ctgatgctga tgagccattt actgggagtt ctaaaattct gtatcatatc 1560
ataaagggag acagtgaggg acgcctgggg gttgacacag atccccatac caacaccgga 1620
tatgtcataa ttaaaaagcc tcttgatttt gaaacagcag ctgtttccaa cattgtgttc 1680
aaagcagaaa atcctgagcc tctagtgttt ggtgtgaagt acaatgcaag ttcttttgcc 1740
aagttcacgc ttattgtgac agatgtgaat gaagcacctc aatttcccca acacgtattc 1800
caagcgaaag tcagtgagga tgtagctata ggcactaaag tgggcaatgt gactgccaag 1860
gatccagaag gtctggacat aagctattca ctgaggggag acacaagagg ttggcttaaa 1920
attgaccacg tgactggtga gatctttagt gtggctccat tggacagaga agccggaagt 1980
ccatatcggg tacaagtggt ggccacagaa gtaggggggt cttccttaag ctctgtgtca 2040
gagttccacc tgatccttat ggatgtgaat gacaaccctc ccaggctagc caaggactac 2100
acgggcttgt tcttctgcca tcccctcagt gcacctggaa gtctcatttt cgaggctact 2160
gatgatgatc agcacttatt tcggggtccc catttacat tttccctcgg cagtggaagc 2220
ttacaaaacg actgggaagt ttccaaaatc aatggtactc atgcccgact gtctaccagg 2280
cacacagact ttgaggagag ggcgtatgtc gtcttgatcc gcatcaatga tgggggtcgg 2340
ccacccttgg aaggcattgt ttctttacca gttacattct gcagttgtgt ggaaggaagt 2400
tgtttccggc cagcaggtca ccagactggg atacccactg tgggcatggc agttggtata 2460
ctgctgacca cccttctggt gattggtata atttagcag ttgtgtttat ccgcataaag 2520
aaggataaag gcaaagataa tgttgaaagt gctcaagcat ctgaagtcaa acctctgaga 2580
agctgaattt gaaaaggaat gtttgaattt atatagcaag tgctatttca gcaacaacca 2640
tctcatccta ttacttttca tctaacgtgc attataattt tttaaacaga tattccctct 2700
tgtcctttaa tatttgctaa atatttcttt tttgaggtgg agtcttgctc tgtcgcccag 2760
gctggagtac agtggtgtga tcccagctca ctgcaacctc cgcctcctgg gttcacatga 2820
ttctcctgcc tcagcttcct aagtagctgg gtttacaggc acccaccacc atgcccagct 2880
aatttttgta tttttaatag agacggggtt tcgccatttg gccaggctgg tcttgaactc 2940
ctgacgtcaa gtgatctgcc tgccttggtc tcccaataca ggcatgaacc actgcaccca 3000
cctacttaga tatttcatgt gctatagaca ttagagagat ttttcatttt tccatgacat 3060
ttttcctctc tgcaaatggc ttagctactt gtgttttcc cttttggggc aagacagact 3120
cattaaatat tctgtacatt ttttctttat caaggagata tatcagtgtt gtctcataga 3180

actgcctgga ttccatttat gtttttctg attccatcct gtgtcccctt catccttgac 3240
tcctttggta tttcactgaa tttcaaacat ttgtcagaga agaaaaaagt gaggactcag 3300
gaaaaataaa taaataaaag aacagccttt tgcggccgcg aattc 3345

<210> 4
<211> 1924
<212> DNA
<213> human

<400> 4

ccatgacgcc cgccctcaca gccctgctct gccttgggct gagtctgggc cccaggacgc 60
gcatgcaggc agggcccttc cccaaaccca ccctctgggc tgagccaggc tctgtgatca 120
gctgggggag ccccgtgacc atctggtgtc aggggagcct ggaggcccag gagtaccaac 180
tggataaaga gggaagccca gagccctggg acagaaataa cccactggaa cccaagaaca 240
aggccagatt ctccatccca tccatgacac agcaccatgc agggagatac cgctgccact 300
attacagctc tgcaggctgg tcagagccca gcgatcccct ggagctggtg atgacaggat 360
tctacaacaa acccaccctc tcagccctgc ccagccctgt ggtggcctca gggggaata 420
tgaccctccg atgtggctca cagaagggat atcaccattt tgttctgatg aaggaaggag 480
aacaccagct cccccggacc ctggactcac agcagctcca cagtgggggg ttccaggccc 540
tgttccctgt gggccccgtg acccccagcc acaggcgtgt ctaggaagcc ctccctcctg 600
accctgcagg gccctgtcct ggcccctggg cagagcctga ccctccagtg tggctctgat 660
gtcggctacg acagatttgt tctgtataag gagggggaac gtgacttcct ccagcgccct 720
ggccagcagc ccccaggctgg gctctcccag gccaacttca ccctgggccc tgtgagccgc 780
tcctacgggg gccagtacag gtgctatggt gcacacaacc tctcctccga gtggtcggcc 840
cccagtgacc ccctggacat cctgatcaca ggacagatct atgacaccgt ctccctgtca 900
gcacagccgg gccccacagt ggcctcagga gagaacatga ccctgctgtg tcagtcacgg 960
gggtattttg acactttcct tctgaccaaa gaaggggcag cccatccccc actgcgtctg 1020
agatcaatgt acggagctca taagtaccag gctgaattcc cccatgagtcc tgtgacctca 1080
gcccacgcgg ggacctacag gtgctacggc tcacgcagct ccaaccccca cctgctgtct 1140
ttccccagtg agcccctgga actcatggtc tcaggacact ctggaggctc cagcctccca 1200
cccacagggc cgccctccac acctggtctg ggaagatacc tggaggtttt gattggggtc 1260
tcggtggcct tcgtcctgct gctcttcctc ctcctcttcc tcctcctccg acgtcagcgt 1320
cacagcaaac acaggacatc tgaccagaga aagactgatt tccagcgtcc tgcaggggct 1380
gcggagacag agcccaagga caggggcctg ctgaggaggt ccagcccagc tgctgacgtc 1440
caggaagaaa acctctagcc cacacgatga agaccccccag gcagtgacgt atgccccggt 1500

```
gaaacactcc agtcctagga gagaaatggc ctctcctccc tcctcactgt ctggggaatt   1560
cctggacaca aaggacagac aggtggaaga ggacaggcag atggacactg aggctgctgc   1620
atctgaagcc tcccaggatg tgacctacgc ccagctgcac agcttgaccc ttagacggaa   1680
ggcaactgag cctcctccat cccaggaagg ggaacctcca gctgagccca gcatctacgc   1740
cactctggcc atccactagc ccggggggta cgcagacccc acactcagca gaaggagact   1800
caggactgct gaaggcacgg gagctgcccc cagtggacac cagtgaaccc cagtcagcct   1860
ggacccctaa cacagaccat gaggagacgc tgggaacttg tgggactcac ctgactcaaa   1920
gatg                                                                1924
```

<210> 5
<211> 1536
<212> DNA
<213> human

<400> 5

```
gtgacgcgag gctctgcgga gaccaggagt cagactgtag gacgacctcg ggtcccacgt     60
gtccccggta ctcgccggcc ggagcccccg gcttcccggg gccgggggac cttagcggca    120
cccacacaca gcctactttc caagcggagc catgtctggt aacggcaatg cggctgcaac    180
ggcggaagaa aacagcccaa agatgagagt gattcgcgtg ggtacccgca agagccagct    240
tgctcgcata cagacggaca gtgtggtggc aacattgaaa gcctcgtacc ctggcctgca    300
gtttgaaatc attgctatgt ccaccacagg ggacaagatt cttgatactg cactctctaa    360
gattggagag aaaagcctgt ttaccaagga gcttgaacat gccctggaga agaatgaagt    420
ggacctggtt gttcactcct tgaaggacct gcccactgtg cttcctcctg gcttcaccat    480
cggagccatc tgcaagcggg aaaaccctca tgatgctgtt gtctttcacc caaaatttgt    540
tgggaagacc ctagaaaccc tgccagagaa gagtgtggtg ggaaccagct ccctgcgaag    600
agcagcccag ctgcagagaa agttcccgca tctggagttc aggagtattc ggggaaacct    660
caacacccgg cttcggaagc tggacgagca gcaggagttc agtgccatca tcctggcaac    720
agctggcctg cagcgcatgg gctggcacaa ccgggtgggg cagatcctgc accctgagga    780
atgcatgtat gctgtgggcc agggggcctt gggcgtggaa gtgcgagcca aggaccagga    840
catcttggat ctggtgggtg tgctgcacga tcccgagact ctgcttcgct gcatcgctga    900
aagggccttc ctgaggcacc tggaaggagg ctgcagtgtg ccagtagccg tgcatacagc    960
tatgaaggat gggcaactgt acctgactgg aggagtctgg agtctagacg gctcagatag   1020
catacaagag accatgcagg ctaccatcca tgtccctgcc cagcatgaag atggccctga   1080
ggatgaccca cagttggtag gcatcactgc tcgtaacatt ccacgagggc cccagttggc   1140
tgcccagaac ttgggcatca gcctggccaa cttgttgctg agcaaaggag ccaaaaacat   1200
```

```
cctggatgtt gcacggcagc ttaacgatgc ccattaactg gtttgtgggg cacagatgcc 1260
tgggttgctg ctgtccagtg cctacatccc gggcttcagt gccccattct cactgctatc 1320
tggggagtga ttaccccggg agactgaact gcaggcttca agccttccag ggatttgcct 1380
caccttgggg ccttgatgac tgccttgcct cctcagtatg tgggggcttc atctctttag 1440
agaagtccaa gcaacagcct ttgaatgtaa ccaatcctac taataaacca gttctgaagg 1500
taaaaaaaaa aaaaaaaaaa aaaaaaaaaa aaaaaa 1536
```